Europäisches Patentamt

European Patent Office

Office européen des brevets

(11) Publication number: **0 153 190 B1**

# EUROPEAN PATENT SPECIFICATION

(45) Date of publication of patent specification: 03.05.89    (51) Int. Cl.⁴: **A 61 B 17/42**

(21) Application number: 85301118.7

(22) Date of filing: 20.02.85

(54) Endoscopic ovum picker instruments.

(30) Priority: 20.02.84 JP 30075/84
24.02.84 JP 35022/84

(43) Date of publication of application:
28.08.85 Bulletin 85/35

(45) Publication of the grant of the patent:
03.05.89 Bulletin 89/18

(84) Designated Contracting States:
AT BE CH DE FR GB IT LI NL SE

(56) References cited:
EP-A-0 072 689
WO-A-82/00754
WO-A-85/00009
US-A-4 369 768

(73) Proprietor: OLYMPUS OPTICAL CO., LTD.
43-2, 2-chome, Hatagaya Shibuya-ku
Tokyo 151 (JP)

(72) Inventor: Shishido, Yoshio
No. 2-1-30-512 Fuchinobe
Sagamihara-shi Kanagawa-ken (JP)

(74) Representative: Kennedy, Victor Kenneth et al
G.F. REDFERN & CO. Marlborough Lodge 14
Farncombe Road
Worthing West Sussex BN11 2BT (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to endoscope means as described in the preamble of Claim 1 or Claim 4, but incorporating a channel intended for an ovum picker that is inserted through said channel for picking up ovum from an ovarian follicle without injuring it, whilst providing for visual observation through said endoscope means to be maintained throughout the operation.

Since the first success in achieving birth of an externally fertilized baby, by Steptoe in the United Kingdom in 1978, interest in external fertilization has been increasing all over the world, including Japan, and the clinical application has been tried in various facilities. There have been reported about 400 cases of birth of externally fertilized babies.

In order to effect external fertilization, it is necessary to pick up the ovum from the mother. As reported by Mr Suzuki et al of the Medical Department of Tohoku University, in the Journal of the Japanese Sterilization Society, No. 4, Vol. 28, 1983, the ovum has been picked up by sucking it through a suction needle inserted through a hole formed through abdominal skin whilst under observation through an abdominal endoscope inserted through another hole pierced through the abdominal skin.

Conventional aspirator needles, as employed for picking up an ovum, are formed as a hollow needle for a syringe, with an inner diameter of about 1.0 mm for picking up the ovum, outer diameter of about 2.0 mm and a length of about 200 to 250 mm. It has sharp edge at the top thereof for piercing the ovarian follicle grown at the surface of the ovary so that the precious ovum which had been carefully protected during long menstruation frequently has been injured by the sharp needle edge during the piercing process.

Moreover, if slight damage caused by the piercing of the needle is not detected, and the fertilization succeeds, a defective human or animal baby will be born. Hence, the utmost care should be exercised in picking up an ovum, the piercing with the needle, external fertilization, implantation and pregnancy.

For the picking up of ovum, the needle must be pierced through the ovarian follicle. The sharp edge of the aspirator needle tip in said conventional practice should be pierced deeply, after the piercing for aspirating and picking up the ovum, with the result in possible damage to the ovum in the ovarian follicle. Particularly, when such an operation is to be performed on a human being, the picking up of ovum is very dangerous.

Moreover, when a fine hole or opening is to be provided for aspirating and picking up ovum and the aspirator needle tip is protruded, the ovarian follicle is displaced, making the formation of a suitable fine hole difficult, and piercing may lead to damage of the inner ovum by the sharp edge. However, said conventional aspirator needle has an advantage in that the ovarian follicle is easily displaced to be pierced in a desired site.

Nevertheless, since said conventional aspirator needle necessitates not only a pierced hole for the abdominal endoscope but also a pierced hole through the abdominal wall for picking up the ovum the needle is not favourable for a patient or an animal. In addition, said conventional aspirator needle has the disadvantage that the aspirator needle for picking up the ovum is independent of the abdominal endoscope and has to be manipulated independently, reducing the workability.

An ovum picking up tool for the external fertilization is claimed in the West German Utility Model No. 76 15 524. An endoscope incorporating a channel is claimed, for example in the US Patent No. 4 252 122 and the West German Utility Model No. G81 04 329.5.

An arthroscope having the combination of features set out in the preamble of Claim 1 is described in the US Patent Specification No. 4 369 768.

One object of the present invention is to provide endoscope means wherein the inlet mouth of channel through the endoscope is partitioned into a plurality of compartments so as to permit the insertion of a plurality of tools, such as a tool for picking up ovum and a clamping forceps concurrently through the channel, so that the clamping forceps may clamp the ovarian follicle firmly when said tool for picking up the ovum is pierced through the ovarian follicle to effect the manipulation for picking up the ovum safely and accurately.

Another object is to provide endoscope means wherein the endoscope means and tool for picking up the ovum can be inserted integrally through the abdominal wall by providing a single piercing hole therethrough, the ovum within the ovarian follicle can be picked up under the visual observation through said endoscope means, thereby mitigating the pain of the patient and improving the operability by surgeons.

A further object is to provide a tool for picking up the ovum wherein the aspirator needle or capillary tube is designed to be movable through the sheath tube for aspirating and picking up the ovum from the ovarian follicle and to be fixative with the sheath tube by manipulating the needle at the handle end to pick up the ovum safely and efficiently without injuring the ovum, operated by a single operator or a small number of operators.

In accordance with one aspect of the invention there is provided an endoscope ovum picker instrument incorporating the features set out in the characterising part of Claim 1.

The invention will now be described with reference to the drawings, in which:—

Figure 1 shows a plan view of a first exemplary embodiment of an abdominal endoscope or laparascope;

Figure 2 is an enlarge front view of the abdominal endoscope of Figure 1;

Figure 3 is enlarged sectional view of the handle of the abdominal endoscope of Figure 1;

Figure 4 shows pressure welded member forming a clamping and fixing means for use with the abdominal endoscope;

Figure 5 is a sectional view of the clamping and fixing means taken along line A—A Figure 3;

Figure 6 shows the tool for picking up ovum, used in the first embodiment;

Figure 7 is an enlarged view of the aspirator needle details in Figure 6;

Figure 8 shows a sectional view of the rear end of the aspirator needle shown in Figure 6;

Figure 9 is a longitudinal section illustrating the structure of stop-cock for the aspirator needle;

Figure 10 is a plan view of the stop-cock shown in Figure 9;

Figure 11 illustrates a pressure welded member formed at the aspirator needle for the clamping and fixing means;

Figure 12 shows a cross-section of the clamping and fixing means for the aspirator needle;

Figure 13 schematically illustrates picking up an ovum by means of the first embodiment;

Figure 14 illustrates a second embodiment of an aspirator needle for picking up ovum;

Figure 15 is an enlarged view of the aspirator tool of Figure 14;

Figure 16 is a section illustrating the structure of a stop-cock for the aspirator tool of Figure 14;

Figure 17 is a side view of the stop-cock; and

Figure 18 shows an aspirator needle inserted from the rear end of said aspirator tool.

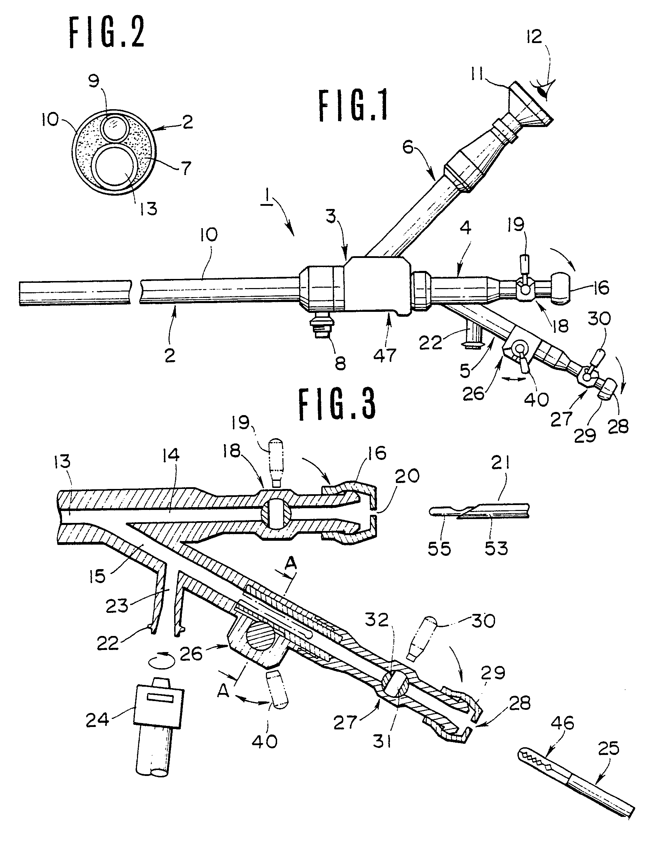

Figures 1 to 13 relate to a first exemplary embodiment of this invention. As can be seen from Figure 1, an abdominal endoscope 1 comprises a rigid, elongate insertion sheath 2 extending from the front longitudinal end of a control body member 3 having a larger diameter or cross-section. From the rear end of the control member 3, a first channel 4 extends in axial alignment with the insertion sheath 2, and a second channel 5 branches obliquely back and down from the first channel 4 at the rear end of the control body member 3, whilst an eyepiece member 6 extends obliquely back and upwards from the upper side of the control body member 3.

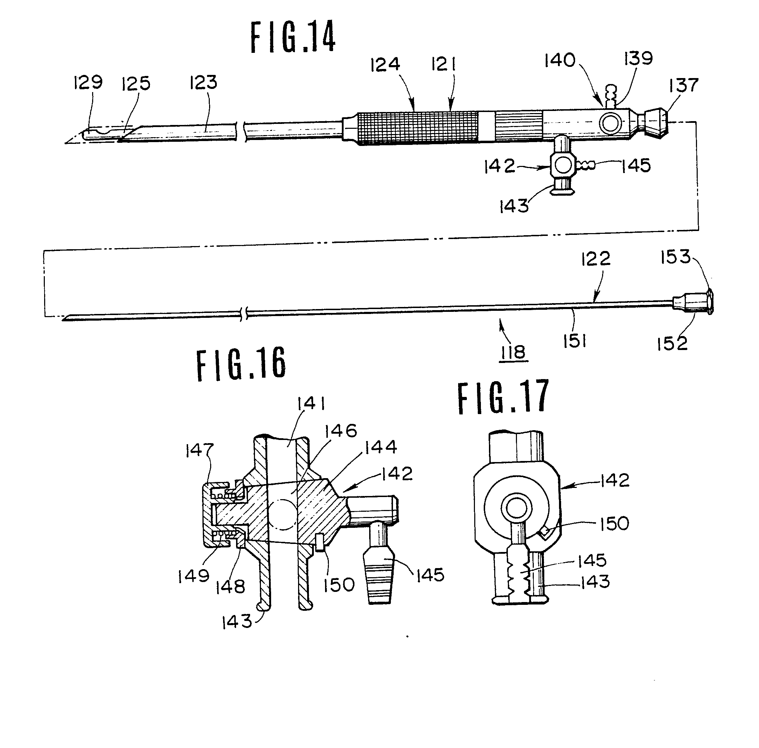

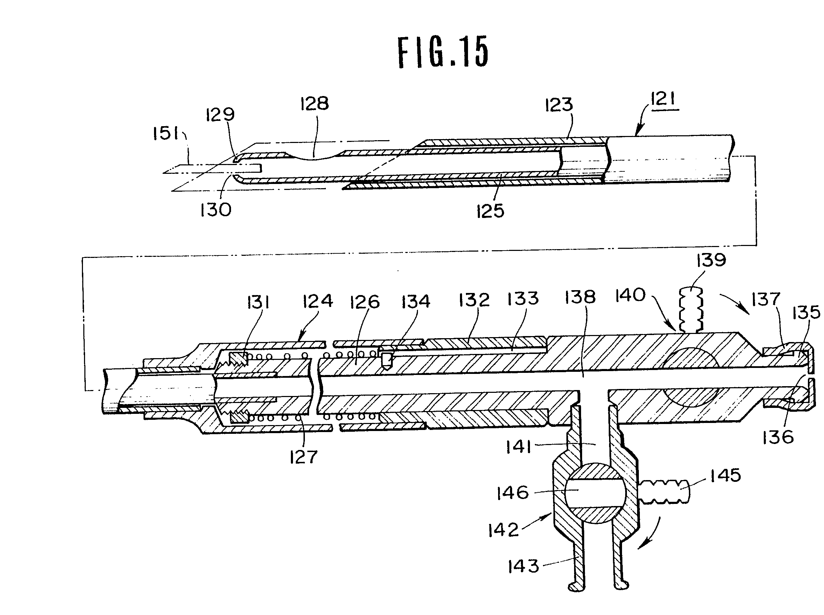

The insertion sheath 2 of said abdominal endoscope contains a light guide 7 comprising a bundle of very fine optical fibres for transmitting illuminating light, bent within the control body member 3 to be fixed to a light guide entry port 8. Illuminating light is transmitted from a light source (not shown) through a light cable (not shown) coupled to said entry port 8, and on through the light guide 7 to be emitted through the leading end face of the light guide to project onward to irradiate a forward zone (i.e. an organ) as shown in Figure 13. The end face of the insertion sheath 2 also carries a sealed tube 9 receiving the observation optical system of the endoscope, which is inserted through the eccentrically arranged tube 9 in the upper part of the outer housing 10 of the sheath 2. The front end of this viewing tube 9 is sealed by a cover glass and receives an objective lens system, relay lens system or image guide for transmitting the image focused by said objective lens system to the eyepiece member 6, branched from the control body member 3 through the observation optical system and eyepiece lens system (not shown) to an eyepiece 11 for the magnified image to be observed by the eye 12 of an operator.

A further hollow pipe extends through said insertion sheath 2 eccentrically below, and adjacent the viewing tube 9, to define a forceps channel 13 in which can pass an operating tool or the like. As shown enlarged in Figure 3, this forceps channel 13 is connected respectively with a hollow passage 14 within the first channel 4 and a hollow passage 15 in the second channel 5 each branched at the rear portion of the control body member 3.

The passage 14 within said first channel 4 is connected with said channel 13 in alignment therewith and carries a rubber cap 16 at its rear end, an intermediate stop-cock 18 being provided midway along the passage 14, and by turning a lever 19 in the direction of an arrow (Figure 1) can open the passage, or close it by turning the lever 19 back to the illustrated position.

The rubber cap 16 has a hole 20 having a size smaller than the outer diameter of a tool 21 for picking up ovum, so as to provide an airtight seal when tool 21 is inserted forwards through said hole 20 and opened stop-cock 18, as indicated in Figure 3.

Said second channel 5, branched from said first channel 4 to extend obliquely downwards, is provided with a mouthpiece 22 protruding down at a point slightly downstream from the branching position, and passage 15 communicates with passage 23 within this mouthpiece 22 so as to feed a veress gas from a veress cannula (not shown) via a connector 24 coupled to said mouthpiece 22.

Clamping and fixing means 26 is provided within said second channel 5 at a downstream position from the position provided with said mouthpiece 22 so as to firmly grip clamping forceps 25 when these are inserted through passage 15 within channel 5. A stop-cock 27 is provided downstream from said clamping means 26, and the insertion port positioned downstream from said stop-cock 27 at the end of the channel 5 is covered by a rubber cap 29 provided with a small central hole 28.

The stop cock 27 is designed to close or open the passage 15 by pivotting a plug 32 that is provided with an open passage 31 by manipulating a control lever 30, and is thus of a similar structure to the stop-cock 18 provided within the first channel 4, described above.

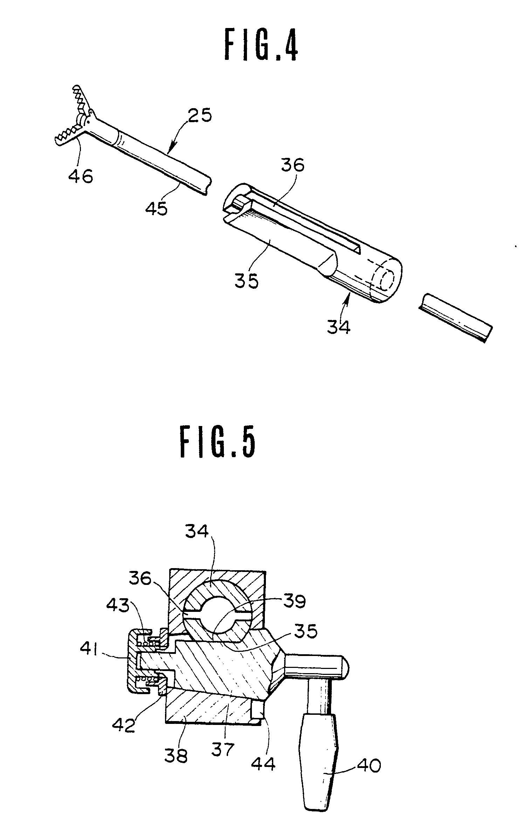

Said clamping and fixing means 26 have a structure as illustrated by Figures 4 and 5.

Namely said means 26 comprise a contact member 34 incorporated within channel 5 for defining a portion of passage 15, and a compression member 37 for clamping on forcepts 25 when inserted through the hollow portion of said pressure contact member 34, by pressing on a flat member 35, formed for example by notching the outer periphery of said contact member 34 lengthwise to an appropriate length, and so reducing the width of a split groove 36 formed by an axial notch so that said split groove is parallel to said flat member 35 or the like. Compression member 37 is formed, for example, by a semi-hard rubber or plastic material, and is received through the tapered through hole in the main body 38 of the clamping and fixing means 26 as shown in Figure 5. The outer tapered periphery of said compression member 37 firmly contacts said tapered through hole and is notched to form a flat recess 39 to engage with the flat member 35 of said contact member 34, as shown in Figure 5. Under such a condition, the contact member 34 has predetermined normal internal diameter to permit an inserted clamping forceps 25 to slide axially. When lever 40 provided at one end of compression member 37 is pivotted, the flat member 35 is turned from the position in which the recess or notched portion 39 firmly contacts the flat member 35 of contact member 34, to the condition that flat member 35 of compression member 34 is pressed against the tapered portion having no recess, at the corner of the recess 39 to reduce the width of the split groove 36 and so reduce the internal effective diameter to press and grip the shaft of the clamping forceps inserted through the inner portion over a wide contact area, to fix firmly the forceps so that they cannot slide axially.

The other end of the compression member 37 remote from the lever 40 is machined stepwise to a small diameter, and covered by a threaded cap nut 41. Said cap nut is biased away from the compression member 37 by a spring 43 between said nut and a washer member 42, so as to keep the contruction substantially air and water tight, a small amount of intervening grease being applied between the outer tapered ground periphery of the compression member 37 and the inner tapered ground periphery of the through hole. Moreover, a pin 44 is provided, protruding from the outer periphery of the compression member 37 to act as a stopper controlling the pivotal angle of the lever 40.

The clamping forceps 25 inserted in the second channel 5 provided with said clamping and fixing means 26 at an intermediate point, have a wire (not shown) inserted through the elongate flexible and hollow shaft 45 so that an alligator-mouth clamp 46 is provided at the tip of the shaft 45 as shown in Figure 4 is opened, for example, by manipulating the wire by a control (not shown) at the operating handle end, and closed to clip the desired objective organ by adjusting said wire.

A handgrip member 47 for easily gripping the control body member 3 is provided by flattening the portion to the rear of the light guide mouthpiece 8 on the face remote from the eyepiece 11.

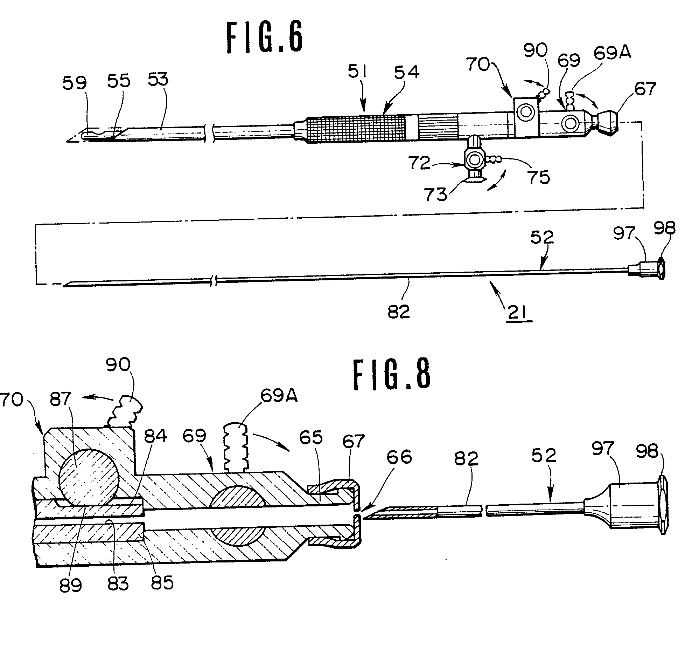

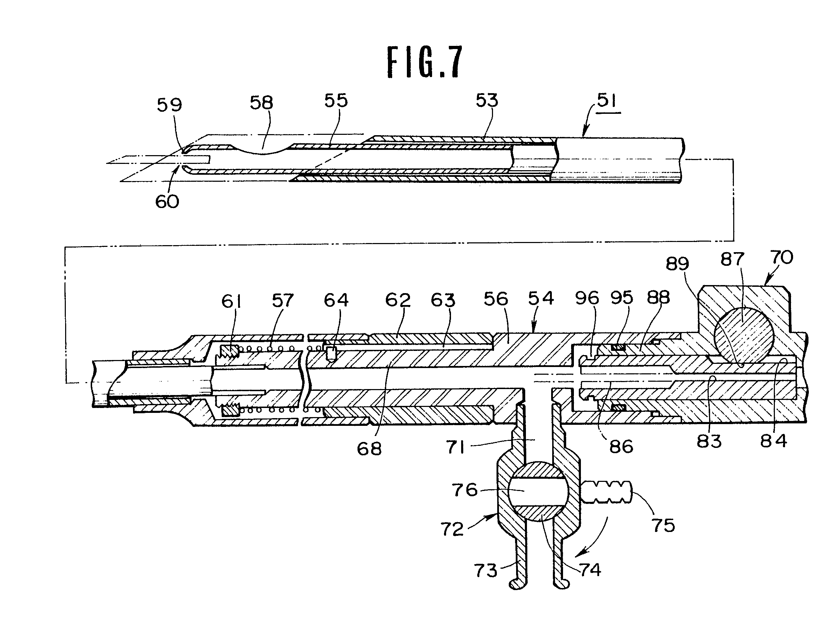

A tool 21 for picking up ovum is inserted freely through the first channel 4 of the abdominal endoscope 1 as shown in Figure 6 to 8, and comprises a picking up needle 51, and hollow aspirator needle 52 capable of being inserted through the inner hollow passage of said picking up needle 51, providing means for aspirating, and for picking up ovum.

Said picking up needle 51 comprises an elongate sheath tube 53 to be insertable from the inserting mouth at the exposed end of first channel 4, through the forceps channel 13 in the abdominal endoscope, extending from an operative member 54 formed by firmly securing a tubular or cylindrical member having a larger diameter at the trailing end of said sheath tube 53 and provided with a knurled pattern for clamping and handling the sheath tube easily, and a fine hollow tube 55 slidable in the axial, longitudinal direction within said sheath tube.

Said fine hollow member 55, within said sheath tube 53, is secured at the trailing end thereof, to the leading end inner periphery of a thick-walled and substantially tubular handle member 56, and the fine hollow member 55 biased axially forwards together with the handle member 56 by means of a spring 57 fitted in a clearance defined between the outer periphery of the front half of the handle member 56 and the surrounding inner periphery of the cylindrical operative member 54 as shown in Figure 7. In short, as shown in Figure 6 or Figure 7, the tip of the fine hollow member 55 is protruded to an appropriate length from the tip of the tube 53, which is formed into sharp edge by cutting the hollow sheath member obliquely. The tip of said fine hollow member 55 is provided with a hole, i.e. side hole 58, so as to irrigate a gas or physiological sodium chloride solution therethrough. Additionally a convergent tip 59 having part-spherical or rounded shape is formed at the front of said side hole 58. A fine hole 60 is pierced through the converged tip so that when the aspirator needle 52 is inserted along the central axis of the fine hollow member 55 via the hole 66 it protrudes through this small hole 60 at the inserted end of the endoscope. The spring 57 for biasing said fine hollow member 55 to be protruded through the small hole 60 is controlled by abutting the leading end thereof with the leading end of the handle member, for example with a nut 61 threaded onto the handle member 56, and the trailing end of the spring abuts against the leading end of a cylindrical sliding member 62 connected at the trailing end of the cylindrical member, comprising operative member 54 and telescoped around the outer periphery of handle member 56. Said sliding member 62 is provided with an elongate axial groove 63, for example, on the upper side of the inner periphery fitted through said handle member 56, for receiving a guide pin 64 at a position so that when the tip of the fine hollow member 55 is displaced backwards by pressing, the hollow member 56 is contracted longitudinally and the guide pin 64 also displaced backwards along the groove 63, and when the guide pin 64 abuts against the rear end of the elongate groove 63, the tip 59 of the fine hollow member 55 is positioned within the obliquely cut tip of the sheath tube 53. It should be noted in Figure 6 or Figure 7 that the sheath tube 53 cannot be displaced from the position as shown in the solid line to the position as shown by a chain-dotted line in Figure 7, but when the hollow member 55 is retracted backwards, the tip of the fine hollow member 55 is retracted within the sheath tube 53, as shown by the chain-dotted line in Figure 6.

Moreover, the fine hollow member 55 is normally attached so that said side hole 58 is positioned to protrude from the tip of the sheath tube 53. Such position may be precisely adjusted, for example, by the position of fixing but 61, or alternatively, for example, by protruding another pin forwards from the handle member 56 to the position apart from the leading end of the sliding member 62.

The spring 57 for biasing said fine hollow member 55 so as to protrude the tip 59 of the fine hollow member 55 is designed so that the spring is contracted together with the elastic member comprising handle member 56 by a weak force for abutting the tip 59 of the fine hollow member 55 against the ovarian follicle, and on pressing it further the sharp edge of the sheath tube 53 is protruded for piercing the edge into the follicular membrane.

The latter half portion adjacent to the portion attached with sliding member 62 of handle member 56 has a thick, stepped wall and the trailing end of the handle member is internally machined so as to form a mouthpiece 65 as shown in Figure 8. This mouthpiece 65 is covered with a rubber cap 67 provided with a small central hole 66 in which to insert the aspirator needle 52.

There is provided a stop-cock 69 in the thick-walled portion of the handle member 56, slightly forward from said mouthpiece 65, so that when the aspirator needle 52 is not inserted in the hollow passage 68, the opened passage 68 through mouthpiece 65 can be closed by pivotting a lever 69A.

In addition, there are provided holding and fixing means 70 for securing the aspirator needle 52, when inserted through said hollow passage 68, and in the handle member 56 at a point slightly forward of said stop-cock 69, and a further stop-cock 72 is provided, with a through hole 71 connecting to the hollow passage 68 in the handle member 56 to protrude downwards at a position forward of said holding and fixing means. Said stop-cock 72 has a structure as illustrated, for example, by Figure 7, 9 and 10.

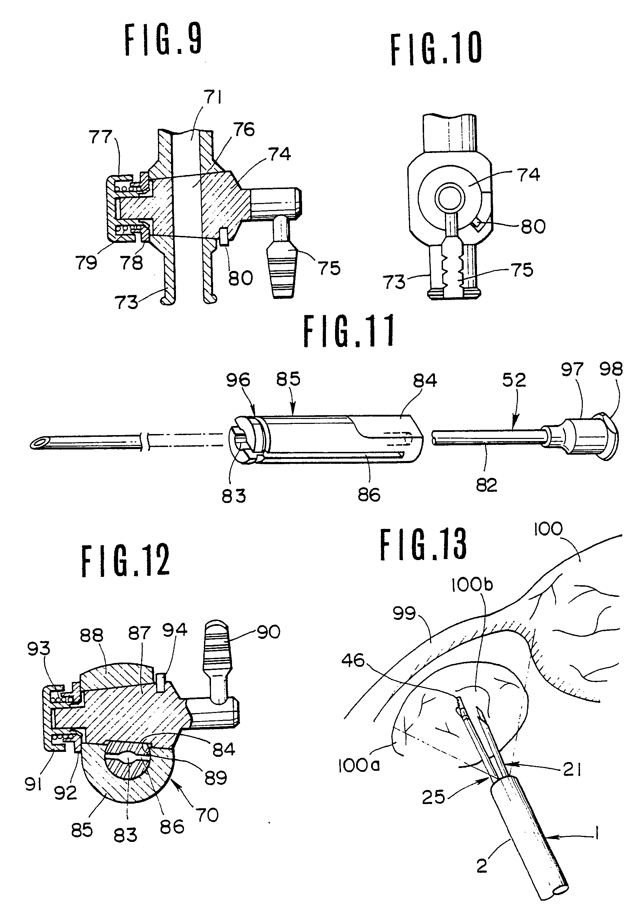

Namely, the through hole or passage 71 within the stop-cocks 72 connects a mouthpiece 73 with the hollow passage 68 of the handle member 56. Stop-cock 72 is provided with a through hole having a diameter larger than said through hole 71, and the hole contains a plug 74. The through hole has a tapered passage therein, and a larger diameter at one end, and the plug 74 is ground down and attached so as to maintain a tightness between the ground surfaces. The plug 74 is tapered at the trailing end and attached with a lever 75 protruding rectangularly at the trailing end, so that the plug 74 is pivoted by actuating the lever 75 for connecting the hole 76 with the through hole 71, as shown in Figures 9 and 10, or for blocking the through hole 71 as shown in Figure 7. The end of the plug 74 protruding in the direction opposite to the end provided with the lever 75 is stepped to a smaller diameter and connected with nut 77 which defines a recess around the outer periphery of the stepped end of said plug 74, for receiving a spring 79 within the recess between a washer member 78 and the nut 77, to urge the nut in the direction opposed to plug 74 by the action of said spring 79 so that the plug is held firmly in contact with the ground portion of the stop-cock 72, to maintain gas and liquid seals.

A pin 80 protrudes from the plug 74 and acts as a stopper for controlling the pivotting range of the lever 75, which is a similar structure to that of the stop-cock 69.

On the other hand, holding and fixing means 70 holds and fixes aspirator needle 52, when inserted forwards through the rubber cap 67, to grip said picking up needle 51 and not allow it to slide axially, and has a structure as shown in Figures 7, 8, 11 and 12.

Namely, said means 70 comprise a contact member 85 having a flat member 84 formed, for example, by notching the outer periphery of the cylindrical or tubular member defining an insertion hole 83 capable of receiving the hollow member 82 of an aspirator needle 52 longitudinally, a compression member 87 for reducing the effective inner diameter of the insertion hole 83 by reducing the width of a split groove 86 formed longitudinally along said contact member 85 by pressing said flat member 84, and a main body 88 of the holding and fixing means 70 comprising a housing for receiving said contact member 85 and compression member 87.

Said compression member 87 is formed into a frustrum of a cone ground to fit through the inner wall of a tapered through hole formed through the main body 88 of the holding and fixing means. A recess 89 on the conical peripheral surface is held in firm contact with the inner wall surface of said through hole, for example, by notching. The inner bottom surface of said recess 89 is flattened so as to engage against said flat member 84 of the contact member 85, as shown in Figures 7 or 12. Under the fitted condition, the inner diameter of the through hole 83 of the contact member 85 is such that the aspirator needle 52 can be slidably inserted axially and means 70 can hold and fix the aspirator needle 52 by pivotting the lever 90 to press upon the flat member 84 of the contact member 85, which has been closed by contact with the tapered corner portion of recess 89, thereby reducing the width of the split groove 85 of the contact member 85 and reducing the effective inner diameter of the through hole 83. The hollow member 82 of the aspirator needle 52 is prevented from deformation or collapse by pressing and fixing the aspirator needle 52 via the contact member 85 provided with its split groove 86, in lieu of direct compression and fixation, thereby increasing the area of contact with the aspirator needle 52. Such compression and fixation can be relieved easily by pivotting the lever 90.

That end of the compression member 87 remote from the end attached to the lever 90 is stepped to a smaller diameter as shown in Figure 12, and threaded to carry a cap nut 91 that is provided to hold the ground tapered portions of the compression member 87 and the surrounding inner wall surface in close contact and maintain a gas tight, sealed hydraulic fit, the nut 91 being biased in the direction opposed to compression member 87 by means of a spring 93 between the nut 91 and a washer member 92. A protruding pin on the outer periphery of the compression member 87 acts as a stopper controlling the pivotting angle of the lever 90.

The main body 88 of the holding and fixing means receives said contact member 85 and, as shown in Figure 7, the outer periphery of the main body 88 is provided with a recess around the trailing end for fitting an O-ring 95 therearound, and for inserting firmly through the machined inner periphery of the handle member 56 at the trailing end thereof to maintain a gas tight hydraulic seal. In addition, a groove 96 is formed around the outer periphery of said contact member 85 at the vicinity of the leading end, to facilitate gripping with the fingers when dissasembling a contact member 85 from a compression member 87. Moreover, the inserting hole 83 is machined around the leading portion from flat member 84 of contact member 85 so as not to contact the inner periphery of hole 83 excessively with the outer periphery of the hollow member 82 of the aspirator needle 52 and to minimize the friction of sliding the hollow member 82 when an inserted aspirator needle is not held pressed to be fixed in position.

On the other hand, the aspirator needle 52 inserted through said picking up needle 51 comprises the elongate hollow member 82 and a handle member 97 having a larger diameter at the rear. A mouthpiece 98 at the trailing end of said handle member enables it to be connected with an aspirator such as syringe or the like by means of a tube or the like.

The operation of this first embodiment of an abdominal endoscope for picking up an ovum will now be described with reference to Figure 13.

If an alveolar ovarian follicle has grown on an ovary surface, the abdominal wall can be provided with a guide hole by piercing it with a trocar. The insertion sheath 2 of an abdominal endoscope 1 can be inserted through said guide hole and veress gas infused through the mouthpiece 22 on the second channel 5, facilitating the insertion of abdominal endoscope 1 and the observation and subsequent operation for picking up the ovum. The fallopian tube 99 connected to the uterus 100 and ovary 100a are observed by means of the optical system of the inserted abdominal endoscope 1. If the presence of an alveolar ovarian follicle 100b on the ovary surface is confirmed, clamping forceps 25 are inserted through the second channel 5 of the abdominal endoscope 1, to pass through passage 15, and a picking up needle 51 is inserted through the first channel 4 to pass through passage 14.

Under observation by the abdominal endoscope 1, supported by one hand of the operator, the vicinity of grown ovarian follicle 102 is clamped by means of the alligator mouth grip 46 at the tip of the clamping forceps 25, by operating the handle of the clamping forceps 25 that protrude forwards through forceps channel 13 of said abdominal endoscope 1 and locked by the pivotting lever 40 for said clamping and fixing means 26. Compression member 37 presses the flat member 35 of the contact member 34 by pivotal operation of the lever 40 to hold and fix the clamping forceps 25 firmly and non-slidably longitudinally. Accordingly, the operator can then remove his hand from the secured clamping forceps 25 and act to protrude the picking up needle 51 through the leading end of the forceps channel 13 of the first embodiment.

In such a case, the aspirating needle 52 is provisionally inserted from the trailing end of said picking up needle 51 to pass therethrough so that the tip of latter needle is retracted slightly into the tip of the sheath tube 53 and the hollow member 82 of the aspirator needle 52 is fixed by pivotting the lever 90 of the holding and fixing means 70.

Compression member 87 is then pressed on the flat member 84 of the contact member 85 by pivotting said lever 90 to reduce the effective inner diameter of the inserting hole 83 of said contact member 85, and to press the hollow member 82 of the aspirator needle 52 to fix it firmly and non-slidably longitudinally.

Under such a condition, with said aspirator needle 52 fixed, the tip of the picking up needle 51 is abutted against alveolar ovarian follicle 100b that is to be picked up, whilst under observation via the abdominal endoscope 1, and can be pressed gently forwards. The tip of the fine hollow member 55 is then retracted by such pressing, to protrude the sharp edge of the sheath tube 53, thereby forming a small opening. When the opening has been enlarged to a size capable to accept the converged tip of the fine hollow member 55, the piercing by the sharp edge of the sheath tube 53 is stopped, to relieve the pressure of the tip 59 of the fine hollow member 55 against the membrane of ovarian follicle, so that the tip 59 of said hollow member 55 is protruded automatically through the opening to be inserted into the ovarian follicle 100b. In such a case, the vicinity of ovarian follicle 100b is clamped and fixed by the alligator mouthgrip 46 at the tip of the clamping forceps 25 so that ovarian follicle 100b is positively held immobile in position when the protruded tip of said picking up needle 51 presses the ovarian follicle 100b that is to be pierced, safely and correctly at the desired portion, to form an opening for picking up the ovum. In addition, the tip 59 of the fine hollow member 55 inserted through said opening is rounded so as to be inserted without danger of injuring the ovum, even when the tip 59 is inserted to a substantial depth of the ovarian follicle 100b. When the tip 59 of said fine hollow member 55 is to be inserted, biological sodium chloride solution is infused through the mouthpiece 73 formed within the handle member 56 of the picking up needle 51, to maintain the ovarian follicle 100b under non-contracted normal condition or swollen condition, the aspirator needle 52 inserted through the picking up needle 51 and fixed so that the tip of the latter needle is retracted to some extent from the tip of the sheath tube 53, relieved from fixation by the pivotal motion of lever 90 and displaced forwards. When the tip of aspiration needle is protruded through the small hole 60 of the fine hollow member 55 to an appropriate depth into the ovarian follicle 100b, the ovum is aspirated and received together with the saline water within the hollow member 82 by displacing backwards the piston of an aspirator of the syringe type connected via a tube with the mouthpiece 98 formed at the handle member 97 for the aspirator needle 52. The aspirator needle is then withdrawn from the picking up needle and lever 69A of the stop-cock 69 operated.

As mentioned hereinbefore, the ovum can be picked up into the aspirator tube in a simple manner.

According to said first embodiment, holding and fixing means 70 are provided for the aspirator needle 52 so that when the ovum is picked up by inserting the picking up needle 51 through the channel 13 of the abdominal endoscope 1, the picking up needle 51 is inserted through the aspirator needle 52 and can be fixed at an appropriate retracted position from the tip of the aspirator needle 52. Hence, when operation other than aspiration by the aspirator needle 52 is carried out, said first embodiment assures operational safety, in that the aspirator needle 52 is prevented from any displacement to protrude the tip of the aspirator needle to a substantial depth in the ovarian follicle 100b, to possibly injure the ovum or deeper tissue, and the ovum can be aspirated and picked up efficiently within a short period of time only by relieving the fixation and protruding the tip of the aspirator needle 52 slightly forwards. In addition, if there is any possibility of displacement of the aspirator needle 52 when only the aspirator is operated for aspirating and picking up operations, the aspirator needle 52 can be fixed in position at an appropriate depth in the ovarian follicle with ease. In addition, when aspirator needle is fixed, the operator can remove his hand from the fixed aspirator needle 52 and can perform other operations, so that a single operator or a small number of operators can pick up the ovum.

It should be noted that the first embodiment is designed to aspirate and pick up the ovum by inserting the aspirator needle 52 through the picking up needle 51, but means for aspirating and picking up the ovum are not restricted to such a structure, and can be designed so that the means incorporate no aspirator needle 52 and provide no side hole 58, but aspirate and pick up the ovum directly via the small hole 60. In such a case, it is preferred to infuse saline water or the like into the ovarian follicle 100b, and aspirate and pick up the ovum together with the saline water. In such a modification, eliminating aspirator needle 52, the trailing end of the fine hollow member 55 is not fixed by a handle member 56, but extended back to the portion for inserting the aspirator needle 52 and fix the needle by means of holding and fixing means 70. In such a case, the inner diameter of the insertion hole 83 should be compatible with the outer diameter of the fine follow member 55, of course.

In the picking up needle 51 constructed according to said first embodiment, the structure is not restricted to biasing the fine hollow member 55 to protrude it forwards by the action of the spring 57, but may be designed so that the fine hollow member 55 is protruded or retracted manually. Alternatively, the sheath tube 53 may be protruded or retracted from the fine hollow member 55.

In addition, the obliquely cut tip of the sheath tube 53 may be modified so that the furthermost portion of the tip is sharpened and the trailing portion of the tip is rounded, so as to pierce the furthermost portion and to form an opening by rotating the furthermost tip. In such a modification, the length of the furthermost sharpened portion of the tip is adjusted to about the thickness of an ovarian follicle, the sharpened edge cannot then pierce into the ovarian follicle so that the possibility for injuring the ovum can be eliminated substantially completely. Moreover, in such a case, the sharpened edge is limited to a length slightly smaller than the thickness of an ovarian follicle, to cut circularly the follicle to about the rounded portion 59 of fine hollow member 55 or the like and to press the circular portion to provide an opening so as to be inserted into the follicle.

Still further, means for fixing the aspirator needle 52 or the fine hollow member 55 are not limited to those of said first embodiment, but include fixing the contact member 85 by pressing, for example, by a thread. Still further, if the aspirator needle 52 or the like can be fixed in a manner not liable to collapse the aspirator needle 52 or the like, such means can be structured to be pressed directly. For example, if the compression member 87 is made of soft material such as rubber or the like, the aspirator needle 52 or the like can be pressed and fixed directly.

Furthermore, when the aspirator needle 52 is employed as means for aspirating and picking up the ovum, an aspirator such as syringe or the like may be incorporated without providing a mouthpiece 98 at the handle member 97. When the aspirator needle 52 is not employed, the handle side of the fine hollow member can be modified as mentioned above.

A second exemplary embodiment of this invention will now be described with reference to Figures 14 to 18.

The abdominal endoscope to be used in this second embodiment is the same as that shown in Figure 1.

A tool 118 for picking up the ovum is insertable through channel 13 from the rear end of the channel member 4 of said abdominal endoscope 1, and composed of a picking up needle 121, as shown separately in Figure 14, or in Figure 15 on enlarged scale, and a hollow aspirator needle 122 is insertable through the hollow passage within said picking up needle 121.

Said picking up needle 121 is composed of an elongate sheath tube 123 inserted through the forceps channel 13 of the abdominal endoscope 1, with a handle member 124 comprising a tubular or cylindrical member having a larger diameter connected with the rear end of said sheath tube 123, and having the outer periphery thereof formed with a knurled pattern to facilitate grasping it easily, and the fine hollow member 125 is attached slidably axially or longitudinally within said sheath tube 123.

As shown in Figure 15, said fine hollow member 125 within said sheath tube 123 is secured fixedly to the inner periphery of a thick-walled and substantially tubular main handle body 126 having the rear end made of a flexible elastic member at its leading end. Said fine hollow member is biased by the action of a spring 127 fitted around the clearance defined between the forward peripheral surface of the main handle body 126 and the inner periphery of the tubular member forming handle member 124, together with main handle body 126, so as to protrude axially forwards. Namely, as shown in Figures 14 or 15, the tip of the fine hollow member 125 is cut obliquely, so as to protrude from the tip of the sheath tube 123 comprising a sharp edge of appropriate length. The tip side of said fine hollow member 125 is provided with a hole at the side thereof, i.e. side hole 128, so as to infuse gas or physiological sodium chloride solution (referred to hereinafter as saline water). The portion forwards of the side hole 128 is formed into a spherically rounded converged tip 129. There is provided a hole 130 at the furthermost portion of said converged tip, so that an aspirator needle 122 inserted through the central axis of the fine follow member 125 can protrude through said hole 130.

Spring 127 acts to bias said fine hollow member 125 to be protruded therefrom and the leading end is controlled by abutting, for example, against nut 131 threaded at the leading end of the main handle body 126 and the trailing end thereof is controlled by abutting against the leading end of an elastic tubular sliding member 132 connected with the rear end of the tubular member comprising the handle member 124 and fitted around the outer periphery of the main handle body 126 at about the central portion. Said sliding member 132 has an axially elongate groove 133 on the inner periphery of the main handle member 126. A guide pin 134 is received within said elongate groove 133, and secured to the main handle member 126 so that when the tip of the fine hollow member 125 is retracted backwards by pressing it, the main handle member 126 is retracted longitudinally and the guide pin 134 is also displaced backwards within the elongate groove 133 until the guide pin 134 abuts against the furthermost end of the elongate groove 133, when the tip portion 129 of the fine hollow tube 125 is retracted to the inside of the obliquely cut tip of the sheath tube 123. Moreover, in Figures 14 or 15, the sheath tube 123 is not displaced from the position as shown by the solid line to that as shown by a chain-dotted line, but when the fine hollow pipe 125 is retracted backwards, the fine hollow pipe 125 is retracted to the position shown by the chain-dotted line in the inside of the sheath tube 123.

Furthermore, the fine hollow member 125 is normally attached so that the side hole 128 protrudes from the tip of the sheath tube 123. In such a case, the position can be minutely adjusted, for example, by the position of the fixing nut 131. The position can also be adjusted by providing said guide pin 134 or another pin to the main handle body 126 at a forward position from the leading end of the sliding member 132.

Spring 127 acts to bias said fine hollow member 125 to press its tip 129 against the ovarian follicle and the tip 129 together with the elastic member comprising the main handle body 126 is then retracted due to the weak force being overpowered. The sharpened tip of the sheath tube 123 then protrudes in place of the tip portion 129 of fine hollow member 125, so as said sharpened tip pierces the membrane of ovarian follicles.

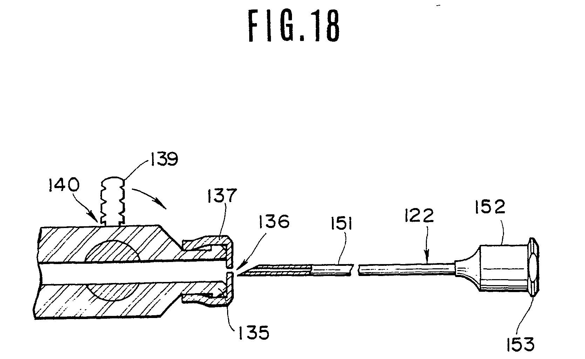

The rear end of said main handle body 126, which is in the vicinity of the portion attached by sliding member 132, is stepped to a larger diameter and the rear end of the stepped thick-walled portion is machined to a small diameter to form a mouthpiece 135, which is covered by a rubber cap 137 provided with a small hole 136 for the insertion of an aspirator needle 122, forwards through said small hole 136, as shown in Figure 18.

There is provided a stop-cock 140 at the thick-walled portion of the main handle body 126 at a position slightly forward of the mouthpiece 135, so that the hollow passage 138 can be closed, and is opened to the mouthpiece 135 when the aspirator needle 122 is to be inserted, the stop-cock being controlled by the pivotal operation of a lever 139.

There is also provided a stop-cock 142 provided with a through hole 141 for connecting the hollow passage 138 of the main handle body 126 to a protruded mouthpiece 143. Said stop-cock 142 has, for example, a structure as shown in Figures 14 to 17.

Namely, a hole or passage 141 in the stop-cock 142 acts to connect the mouthpiece 143 with the hollow passage 138 of the main handle body 126.

The stop-cock 142 has a through hole having a larger diameter than that of said through hole 141, normal to the direction of said through hole 141, and a tapered plug 144 is inserted therein. Said through hole is tapered to conform with the ground surface of the plug 144, said ground, tapered surface acting to maintain the tightness. The plug rear end of larger diameter is machined to a smaller diameter and provided with a protruding lever 145 normal to the plug axis, so that the plug 144 is pivotted by pivotal operation of said lever 145 between a position in which the through hole 146 formed through said plug 144 is connected to the through hole 141, as shown in Figure 16 or blocked from said through hole as shown in Figure 15 or by the chain-dashed line in Figure 16. The opposite end of said plug 144 is stepped to a smaller diameter and secured by a nut 147 with a recess around its outer periphery. A spring 149 is received within the space defined by said recess and a washer member 148, to press the nut 147 in the direction away from the plug 144 by the action of the spring 149, so as to hold the plug 144 closely in contact with the ground surface of the through hole to maintain a gas and liquid tight fit.

A pin 150 protruding from the plug 144 acts as a stopper for controlling the pivotal angle of the lever 145.

Thus, the stop-cocks 140 and 152 have a similar structure.

The aspirator needle 122 which is insertable via the rubber cap 137 of said picking up needle 121 comprises the fine hollow tube 151 inserted through the hollow passage 138 of the picking up needle 121, and the clamping member 152 having a larger diameter and connected with the rear end of said fine hollow tube 151, and the rear end of said clamping member 152 is formed as a mouthpiece 153 capable of connecting an aspirator tool, such as syringe or the like. The tip of said hollow tube member 151 is cut obliquely, such as is the needle tip of a syringe, and the sharpened edge is designed to protrude from the small hole 130 provided at the rounded tip after the sharpened tip of the sheath tube 123 has pierced into the membrane of ovarian follicle so that when the sharp edge of the fine hollow tube 151 is pierced into the membrane of an ovarian follicle, it can pick up the ovum by aspiration without injuring the ovum.

The method of picking up the ovum by means of the abdominal endoscope according to said second embodiment will now be described with reference to Figure 13.

If an alveolar ovarian follicle has grown on the surface of ovary, the abdominal wall of mother's body can be provided with a guide hole by piercing a trocar. The insertion sheath 2 of an abdominal endoscope 1 may then be inserted through said guide hole. A veress gas is infused through the stop-cock mouthpiece 22 provided in the vicinity of the branch of the second channel 5 for facilitating the insertion of laparoscope 1 and observation and subsequent picking up operation. The insertion sheath 2 of the abdominal endoscope 1 is then inserted and the ovary 100a connected to uterus 100 through a fallopian tube 99 is observed via the optical observation system through the eyepiece 11 from the tip of insertion sheath 2, as shown in Figure 13. When the presence of alveolar ovarian follicle 100b is confirmed on the surface of ovary 100a, the clamping forceps 25 is inserted forwards from the rear end of the second channel 5 of the abdominal endoscope 1 through passage 15 and the picking up needle 51 is inserted forwards through the passage 14 within said channel 4 from the rear end of the first channel 4.

The vicinity of the grown ovarian follicle 87 is clamped by the alligator mouthgrip 46 at the tip of the clamping forceps 25 protruded forwards through the channel hole 13 of said abdominal endoscope 1 by manipulating the control handle of the forceps 25 under the observation of the abdominal endoscope grasped by one hand, the clamping forceps 25 locked in the clamped condition. The lever 40 of the holding and fixing means 26 is pivotted to permit the compression member 37 to press the flat member 35 of the contact member 34 provided with the split groove 36 to reduce the effective inner diameter and to hold and fix the clamping forceps 25 inserted through channel hole 13 so as not to be slidable axially. Accordingly, the operator can now remove his hand from the fixed clamping forceps 25 and proceed to effect the operation for protruding the picking up needle 21 forwards from the leading end of the channel hole 13 to bring the tip of the needle 121 against the ovarian follicle 100b to be operated, and press it forwards gently and carefully. Then the protruded tip of the fine hollow member 125 is retracted by the pressure on the picking up needle, to cause the sharp edged tip of the sheath tube 123 to protrude and form a small opening. When said opening has been enlarged to a size capable to allow insertion of the convergent tip of the fine hollow member 125, the piercing of sharp tip of sheath tube 123 is stopped and tip 129 of fine hollow member 125 is protruded automatically to be inserted into ovarian follicle 100b through said opening. Since the vicinity of ovarian follicle 100b is clamped and fixed by means of alligator mouth 46 at the tip of clamping forceps 25, ovarian follicle 100b is prevented from the displacement when the protruded tip of said picking-up needle 121 presses ovarian follicle 100b so that picking-up needle 121 can be pierced safely and correctly through the desired portion to form an opening for picking up the ovum. Moreover, rounded tip 129 of fine hollow member 125 has no possibility for injuring the ovum by inserting the tip into ovarian follicle 100b to a substantial depth, so that the ovum can be aspirated and picked up together with the saline water or veress gas from ovarian follicle 100b maintained under the normal condition or swollen by infusing the saline water or the like through side hole 128 of fine hollow tube 125 from the rounded tip of aspirator needle 122

9

protruded through small hole 130 of said fine hollow member 125. By the way, the ovum is picked up by connecting an aspirator such as a syringe with cock mouthpiece 153 at the trailing handling end of clamping means 152 for aspirator needle 122 by means of a tube and aspirating by said aspirator.

According to said second embodiment, since said abdominal endoscope is attached branchedly with two channels 4, 5 capable to insert clamping forceps 25 in addition to picking-up needle 121, an opening for picking up the ovum can be formed safely and correctly under the condition that the vicinity of portion to be picked up by clamping forceps 25 and the ovum can be picked up conveniently by means of aspirator needle 122 inserted through small hole 130 of fine hollow member 125.

Furthermore, since holding and fixing means 26 is provided within channel 5 for inserting said clamping forceps 25, the forceps can be fixed firmly. Accordingly, the picking-up operation can be done only by one operator, if desired. Moreover, said holding and fixing means 26 don't fix the forceps locally by means of thread, etc., but pinch and fix the forceps over a wide area so that there is no fear to recess or deform such a hollow member.

Still further, in said second embodiment, flexible clamping forceps 25 is inserted through bended second channel 5 and rigid picking-up needle 121 is inserted through straight first channel 4. However, such a mechanism is not limited so that a flexible picking-up needle may be inserted through the bended channel and the rigid clamping forceps through the straight channel which is provided, in turn, holding and fixing means.

The means according to this invention can be employed for picking up not only the human ovum, but also for animal ovum such that of a milch cow, beef cattle, race-horse, etc., which are desired to be bred maintaining a commercially high purity blood strain.

## Claims

1. An endoscope instrument incorporating an elongate insertion sheath (2) insertable through a body cavity, incorporating an observation optical system, an illuminating optical system (7) with an eyepiece (11), and a forceps channel (13) capable to accept and insert an operational tool from the outside via an intermediate control body (3) which carries said insertion sheath and said eyepiece (11) into the body cavity in the axial direction of said elongate insertion sheath, characterised in that branched entry ports (16, 28) are provided at the rear end of the forceps channel whereby a plurality of passages (14, 15) communicate with said forceps channel to permit the concurrent insertion of a number of tools such as an ovum picker and a clamping forceps through said forceps channel, and at least one of said passages being provided with means (26) for holding and securing an operational tool or the like, when inserted therethrough.

2. An endoscope ovum picker as claimed in Claim 1, characterised in that each said means for holding and securing said tool are sealed by a stop-cock (27, 69, 72, 142) and aligned, finely-apertured end-caps (16, 29, 137).

3. An endoscope ovum picker as claimed in Claim 1 or Claim 2, characterised in that the main body member at the rear end of said insertion sheath has a first mouthpiece (16) in alignment with said insertion sheath, a second mouthpiece (28) branched obliquely, and an endoscopic eyepiece (11) attached obliquely backwards, said passages (14, 15) each having stop-cocks and passage (15) having a port for gas or saline fluid.

4. An elongate tool for use in an instrument as claimed in any preceding Claim for picking up the ovum when inserted through a channel of said endoscope, comprising a fine hollow member (55, 125) with a convergent tip (59, 129), provided with a small hole (60, 130) aligned with its channel a sharp-tipped sheath tube (53, 123) telescoping said fine hollow member axially and relatively thereto, an aspirating needle (52, 151) for picking up the ovum capable to be displaced axially within said fine hollow member in said sheath tube so that it can extend through said small hole and capable to pick up the ovum by its aspiration, and means for fixing detachably said needle in said fine hollow member for aspirating and picking up the ovum.

5. A tool as claimed in Claim 4, characterised in that said means for fixing said means for aspirating and picking up the ovum are designed so that the effective diameter of the inserting hole is variable by forming a split groove inside a contact member that embraces said means for aspirating and picking up the ovum, and pressing upon said member to narrow said inserting hole by pivotal motion of a lever.

## Patentansprüche

1. Endoskopisches Instrument mit einer in einen Körperhohlraum einführbaren länglichen Scheide (2) einschließlich eines optisches Betrachtungssystems, mit einem optischen Beleuchtungssystem (7) und einem Okular (11) sowie mit einem Pinzettenkanal (13), durch den über einen Zwischenkörper (3), der die Scheide (10) und das Okular (11) trägt, ein Operationswerkzeug von außen in den Körperhohlraum einführbar ist, dadurch gekennzeichnet, daß verzweigte Eingangsmündungen (16, 28) am hinteren Ende des Pinzettenkanals vorgesehen sind, wodurch mehrere Durchgänge (14, 15) mit dem Pinzettenkanal in Verbindung stehen, um ein im gleichen Zeitintervall ablaufendes Einfügen einer Anzahl von Werkzeugen wie einen Eizellenentnehmer und einer Klemm- oder Spannpinzette durch den Pinzellenkanal zu gewähren, wobei zumindest einer der Durchgänge mit einer Einrichtung (26) zum Halten und Sichern eines Operationswerkzeuges oder dgl. bei dessen Einführung durch ihn hindurch vorgesehen ist.

2. Endoskopisches Instrument nach Anspruch 1, dadurch gekennzeichnet, daß die Einrichtung zum Halten und Sichern des Werkzeuges durch einen Absperrhahn (27, 69, 72, 142) und durch ausgerichtete, mit feinen Öffnungen versehene Endkappen (16, 29, 137) abgedichtet ist.

3. Endoskopisches Instrument nach einem der vorhergehenden Ansprüche 1 oder 2, dadurch gekennzeichnet, daß das Hauptkörperglied am hinteren Ende der Einführungsscheide ein erstes, mit der Einführungsscheide ausgerichtetes Mundstück (16), ein zweites schräg verzweigtes Mundstück (28) und ein endoskopisches Mundstück (11) hat, das schräg nach rückwärts befestigt ist, wobei die Durchgänge (14, 15) jeweils über Absperrhähne verfügen und der Durchgang (15) eine Öffnung für Gas oder Salzflüssigkeit besitzt.

4. Längliches Werkzeug zur Verwendung in einem Instrument nach einem der vorhergehenden Ansprüche 1 bis 3 zur Entnahme von Eizellen während der Einführung durch einen Kanal des Endoskops, gekennzeichnet durch einen feinen Hohkörper (55, 125) mit einer konvergierenden Spitze (59, 129) versehen mit einem kleinem, mit dessen Kanal ausgerichteten Loch (60, 130), ein scharf zugespitzt verlaufendes Scheidenrohr (53, 123), das das feine Hohlglied axial und diesem gegenüber ein- und ausschiebt, eine Ansaugnadel (52, 151) zur Entnahme der Eizelle, die in dem feinen Hohlglied in dem Scheidenrohr axial verschiebbar ist, so daß sie sich durch das kleine Loch hindurch erstrecken und die Eizelle durch Ansaugwirkung entnehmen kann, und eine Einrichtung zum lösbaren Befestigen der Nadel in dem feinen Hohlglied zum Ansaugen und Entnehmen der Eizelle.

5. Werkzeug nach Anspruch 4, dadurch gekennzeichnet, daß die Einrichtung zum Befestigen der Ansaug- und Eizellenentnahmeeinrichtung so ausgebildet ist, daß der wirksame Durchmesser des Einführungsloches variable ist, indem eine Spaltrille innerhalb eines Kontaktgliedes gebildet ist, die die Ansaug- und Eizellenentnahmeeinrichtung umgreift, und auf das Glied gedrückt wird, um das Einführungsloch durch Schwenkbewegung eines Hebels zu verengen.

**Revendications**

1. Instrument d'endoscopie incorporant une gaine d'insertion allongée (2) insérable par une cavité corporelle, incorporant un système optique d'observation, un système optique d'éclairage (7) avec un oculaire (11), et un canal de forceps (13) capable de recevoir et d'insérer un outil d'opération de l'extérieur via un corps de commande intermédiaire (3) qui porte ladite gaine d'insertion et ledit oculaire (11) dans la cavité corporelle dans la direction axiale de ladite gaine d'insertion allongée, caractérisé en ce que des orifices d'entrée ramifiés (16, 28) sont ménagés à l'extrémité arrière du canal de forceps, ce qui fait que plusieurs passages (14, 15) communiquent avec ledit canal de forceps pour permettre l'insertion concurrente d'un certain nombre d'outils comme un appareil de prélèvement d'ovocytes et une pince-forceps à travers ledit canal de forceps, et au moins un desdits passages étant muni d'un moyen (26) pour tenir et fixer un outil d'opération ou analogue lorsqu'on l'y insère.

2. Appareil de prélèvement d'ovocytes pour endoscope selon la revendication 1, caractérisé en ce que chacun des dits moyens pour tenir et fixer ledit outil est fermé par un robinet d'arrêt (27, 69, 72, 142) et un couvercle d'extrémité aligné (16, 29, 137) à ouverture fine.

3. Appareil de prélèvement d'ovocytes pour endoscope selon la revendication 1 ou la revendication 2, caractérisé en ce que l'élément de corps principal à l'extrémité arrière de ladite gaine d'insertion a un premier embout (16) en alignement avec ladite gaine d'insertion, un second embout (28) branché obliquement, et un oculaire endoscopique (11) attaché obliquement vers l'arrière, lesdits passages (14, 15) ayant chacun un robinet d'arrêt et le passage (15) ayant un orifice pour du gaz ou du fluide saline.

4. Outil allongé pour utilisation dans un instrument selon l'une quelconque des revendications précédentes pour prélever l'ovocyte lorsqu'on l'insère par un canal dudit endoscope, comprenant un élément creux et fin (55, 125) avec une extrémité convergente (59, 129) munie d'un petit trou (60, 130) aligné avec son canal, un tube de gaine à bout pointu (53, 123) monté axialement de manière télescopique par rapport audit élément creux et fin, une aiguille d'aspiration (52, 151) pour prélever l'ovocyte pouvant être déplacée axialement dans ledit élément creux et fin dans ledit tube de gaine de manière à pouvoir traverser ledit petit trou et capable de prélever l'ovocyte par son aspiration, et un moyen pour fixer de manière détachable ladite aiguille dans ledit élément creux et fin pour aspirer et prélever l'ovocyte.

5. Outil selon la revendication 4, caractérisé en ce que ledit moyen pour fixer ledit moyen pour aspirer et prélever l'ovocyte est conçu de manière que le diamètre efficace du trou d'insertion soit variable par formation d'une rainure fendue dans un élément de contact qui entoure ledit moyen pour aspirer et prélever l'ovocyte, et pression sur ledit élément pour rétrécir ledit trou d'insertion par le pivotement d'un levier.

# FIG.2

# FIG.1

# FIG.3

# FIG.4

# FIG.5

EP 0 153 190 B1

# FIG.6

# FIG.8

3

# FIG.7

EP 0 153 190 B1

# FIG.9

# FIG.10

# FIG.11

# FIG.12

# FIG.13

# FIG.14

129  125  123  124  121  140  139  137

142  145

143

122  153

118  151  152

# FIG.16

# FIG.17

141

147  146  144  142

149  148  150  145

143

142

150

145

143

# FIG.15

EP 0 153 190 B1

# FIG.18